# FASCICULE DE BREVET EUROPEEN

(11) **EP 2 663 302 B1**
(45) Date de publication et mention de la délivrance du brevet: **20.12.2017**
(21) Numéro de dépôt: 12701947.9
(22) Date de dépôt: 13.01.2012
(51) Int. Cl.: A61K 31/335, A61K 31/366, A61P 35/00

(54) **UTILISATION DE LA SIMALIKALACTONE E COMME AGENT ANTICANCEREUX**
VERWENDUNG VON SIMALIKALACTON E ALS ANTIKREBSMITTEL
USE OF SIMALIKALACTONE E AS AN ANTICANCER AGENT

(30) Priorité: 14.01.2011 FR 1100125
(43) Date de publication de la demande: 20.11.2013
(73) Titulaire: Institut de Recherche pour le Développement, 13572 Marseille Cedex 02 (FR)
(72) Inventeur: BOURDY, Geneviève, F-31520 Ramonville Saint Agne (FR); AUBERGER, Patrick, F-06100 Nice (FR); JULLIAN, Valérie, F-31400 Toulouse (FR); ROBERT, Guillaume, F-06100 Nice (FR); DEHARO, Eric, F- 31190 Auterive (FR)
(74) Mandataire: Gevers & Orès
(86) Numéro de dépôt international: PCT/IB2012/050179
(87) Numéro de publication internationale: WO 2012/095820

(56) Documents cités:
- FR-A1- 2 946 980
- US-A- 5 605 810
- CACHET N ET AL: "Antimalarial activity of simalikalactone E, a new quassinoid from Quassia amara L. (Simaroubaceae)", ANTIMICROBIAL AGENTS AND CHEMOTHERAPY, AMERICAN SOCIETY FOR MICROBIOLOGY, WASHINGTON, DC, US, vol. 53, no. 10, 1 octobre 2009 (2009-10-01), pages 4393-4398, XP008118045, ISSN: 0066-4804, DOI: DOI:10.1128/AAC.00951-09 [extrait le 2009-08-10] cité dans la demande
- "Quassia Tincture from Amazon Herbs", Tropilab Inc , 9 mars 2009 (2009-03-09), XP002646395, Extrait de l'Internet: URL:http://web.archive.org/web/20090309071 437/http://www.tropilab.com/quassiatinctur e.html [extrait le 2011-06-29]
- MUHAMMAD ILIAS ET AL: "A new antimalarial quassinoid from Simaba orinocensis.", JOURNAL OF NATURAL PRODUCTS MAY 2004 LNKD- PUBMED:15165136, vol. 67, no. 5, mai 2004 (2004-05), pages 772-777, XP002646396, ISSN: 0163-3864
- KUMAR V ET AL: "Synthetic medicinal chemistry of selected antimalarial natural products", BIOORGANIC & MEDICINAL CHEMISTRY, PERGAMON, GB, vol. 17, no. 6, 15 mars 2009 (2009-03-15), pages 2236-2275, XP025981922, ISSN: 0968-0896, DOI: DOI:10.1016/J.BMC.2008.10.072 [extrait le 2008-11-05]
- FUKAMIYA N ET AL: "Structure-activity relationships of quassinoids for eukaryotic protein synthesis", CANCER LETTERS, NEW YORK, NY, US, vol. 220, no. 1, 18 mars 2005 (2005-03-18) , pages 37-48, XP004769038, ISSN: 0304-3835, DOI: DOI:10.1016/J.CANLET.2004.04.023
- MURAKAMI C ET AL: "Multidrug-resistant cancer cell susceptibility to cytotoxic quassinoids, and cancer chemopreventive effects of quassinoids and canthin alkaloids", BIOORGANIC & MEDICINAL CHEMISTRY, PERGAMON, GB, vol. 12, no. 18, 15 septembre 2004 (2004-09-15), pages 4963-4968, XP004542852, ISSN: 0968-0896, DOI: DOI:10.1016/J.BMC.2004.06.045
- DATABASE MEDLINE [Online] US NATIONAL LIBRARY OF MEDICINE (NLM), BETHESDA, MD, US; mai 1981 (1981-05), TRAGER W ET AL: "Antimalarial activity of quassinoids against chloroquine-resistant Plasmodium falciparum in vitro.", XP002646397, Database accession no. NLM7020445 & THE AMERICAN JOURNAL OF TROPICAL MEDICINE AND HYGIENE MAY 1981 LNKD- PUBMED:7020445, vol. 30, no. 3, mai 1981 (1981-05), pages 531-537, ISSN: 0002-9637
- ZHANG WEI ET AL: "MAPK signal pathways in the regulation of cell proliferation in mammalian cells", CELL RESEARCH - XIBAO YANJIU, NATURE PUBLISHING GROUP, GB, CN, vol. 12, no. 1, 1 March 2002 (2002-03-01), pages 9-18, XP002525618, ISSN: 1001-0602, DOI: 10.1038/SJ.CR.7290105
- Satoshi Numazawa ET AL: "Regulation of ERK-Mediated Signal Transduction by p38 MAP Kinase in Human Monocytic THP-1 Cells", J. Biochem. The Japanese Biochemical Society, vol. 133, no. 599, 1 January 2003 (2003-01-01), pages 599-605, XP55282001, DOI: 10.1093/jb/mvg077

## Description

L'invention a pour objet l'utilisation de la Simalikalactone E (SkE) comme agent anticancéreux, plus particulièrement pour la prévention et/ou le traitement de cancers épithéliaux et hématologiques.

La Simalikalactone E est un Quassinoïde isolé d'une Simaroubaceae amazonienne, *i.e.* la plante *Quassia amara* réputée pour son efficacité antiparasitaire et sa faible toxicité (Vigneron et al., Journal of Ethnopharmacology (2005), Vol. 98, pp. 351-360 ; Bertani et al., Journal of Ethnopharmacology (2005), Vol. 98, pp. 45-54). La Simalikalactone E a été décrite dans la Demande de brevet FR n°09 02959 pour son activité antipaludique *in vitro* contre *Plasmodium falciparum*, *in vivo* par voie orale contre *Plasmodium* murin, et également contre les formes hépatocytaires et gamétocytaires de ce même parasite chez l'homme. Il a été démontré que la Simalikalactone E inhibe 50% de la croissance de *Plasmodium falciparum* en culture à des doses comprises entre 24 et 68 nM, indépendamment de la sensibilité à la chloroquine de la souche testée. Elle inhibe 50% de la croissance des cellules de mammifère Vero à une dose de 6574 nM, ce qui lui confère un index de sélectivité supérieur à 100. La Simalikalactone E réduit également le taux de gamétocytes de 50% à une concentration sept fois inférieure à celle obtenue avec la primaquine, la molécule de référence pour cette activité. *In vivo*, sur un modèle murin de paludisme, la Simalikalactone E inhibe 50% de la croissance de *Plasmodium vinckei petteri* à des doses de 0,5 et 1 mg/kg/jour par voie intrapéritonéale et par voie orale, respectivement (la chloroquine, molécule de référence, inhibe 50% de la croissance de *Plasmodium vinckei petteri* à une dose de 3 mg/kg/jour par voie intrapéritonéale).

Une étude préclinique chez la souris a par la suite permis d'identifier les doses de Simalikalactone E tolérées : 1 mg/kg/j par voie orale pendant 4 jours dans le test antimalarique suppressif de 4 jours de Peters (Cachet et al., Antimicrobial Agents Chemotherapy (2009) Vol. 53, No. 10, pp. 4393-4398). Cette étude a également démontré une action cytotoxique modérée de la Simalikalactone E sur certaines lignées cellulaires. Toutefois, il n'est fait aucune mention d'un quelconque effet anti-tumoral *in vitro* ou *in vivo* de la Simalikalactone E. Et il convient également de noter que la démonstration d'une activité cytotoxique d'un composé donné même vis-à-vis de lignées cellulaires tumorales n'implique en rien un effet anti-cancéreux, ce composé pouvant s'avérer inactif *in vivo* ou bien présenter une cytotoxicité incompatible avec son administration à un être vivant.

De manière surprenante, les Inventeurs ont pu constater que la Simalikalactone E se révèle être un inhibiteur sélectif de la voie de signalisation Ras/Raf/MEK/Erk, voie de signalisation fréquemment altérée dans les cancers.

La voie de signalisation Ras/Raf/MEK/Erk est activée dans 20% des cancers connus, *i.e.* les cancers du sein, de la prostate, du pancréas, des poumons, de la thyroïde, et dans plus de 50% des cas de leucémies aiguës. Par exemple, la molécule Ras est mutée dans 15% des cancers humains, et plus particulièrement N-ras (Q61L) est muté dans 15 à 30% des mélanomes et B-raf dans 60% des mélanomes et dans 30 à 50% des cancers de la thyroïde.

L'activation de la voie de signalisation Ras/Raf/MEK/ERK est rapidement et clairement identifiable. Une des méthodes pouvant être utilisée pour identifier cette voie est l'analyse de la phosphorylation protéique par une technique de cytométrie en flux ou « phosphoflow » (Firaguay et al., Analysis of signaling events by dynamic phosphoflow cytometry. Sci Signal, 2: p. 13, 2009). Cette technique permet de suivre de manière dynamique le niveau de phosphorylation, et donc l'activation des kinases MEK et ERK.

De plus, des mutations ou translocations bien définies sont associées aux pathologies dans lesquelles la voie Erk1/2 (MAP kinase) est suractivée. Ces mutations connues peuvent être rapidement identifiées chez un patient par pyroséquençage (Borras et al., BMC Cancer 2001, 11 : 406, Lennerz et al., Br. J. Haematol., 7 dec. 2011, DOI : 10.1111/j.1365-2141.2011.08963.x), une technique permettant d'effectuer sans clonage un séquençage rapide, avec une lecture directe de la séquence obtenue, et ce à moindre coût qu'un séquençage par la méthode de Sanger.

La Simalikalactone E a de ce fait un effet plus marqué dans les lignées de cellules cancéreuses dans lesquelles ces voies sont constitutivement activées, telles que les lignées de mélanomes et les hémopathies malignes.

Les Inventeurs ont établi que la Simalikalactone E possède de puissants effets anti-prolifératifs et inducteur de mort cellulaire *in vitro,* plus particulièrement sur les cellules tumorales dans lesquelles la voie Erk1/2 (MAP kinase) est suractivée. Le mécanisme d'action identifié est lié à une inhibition de la voie de signalisation Ras/Raf/MEK/Erk. Le ciblage de la voie Erk1/2 (MAP kinase) est un enjeu majeur en cancérologie, dans la mesure où des mutations activatrices ou des suractivations de cette voie sont couramment retrouvées dans les cancers humains. C'est notamment le cas de B-raf dans le mélanome qui conduit à une suractivation de la kinase Erk1/2 (MAP kinase). De même, dans la Leucémie Myéloïde Chronique (LMC), la tyrosine kinase p210 BCR-ABL codée par le gène issu de la translocation t(9;22), anomalie responsable de la maladie, induit une suractivation de la kinase Erk1/2 (MAP kinase). Par ailleurs, certaines lignées de LMC résistantes aux ITK (Glivec®, Sprycel®) présentent une exacerbation de la kinase Erk1/2 (MPA kinase) due à une augmentation de l'expression et de l'activité des Src kinase Fyn et Lyn (Fenouille et al., Cancer Research (2010), 70 (23): 9659-70 ; Gioia et al., Blood, 2011, 118: 2211-2221).

L'invention a donc pour objet la Simalikalactone E répondant à la formule 1 ci-dessous : 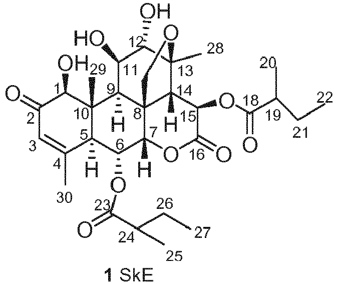 pour son utilisation pour la prévention et/ou le traitement de cancers, et plus particulièrement les cancers épithéliaux et hématologiques dans lesquels la voie de signalisation Ras/Raf/MEK/Erk est activée; la Simalikalactone E inhibant de manière efficace la voie Erk1/2 (MPA kinase), et plus particulièrement la phosphorylation de la kinase Raf et les kinases en aval MEK et Erk.

L'invention a donc pour objet la Simalikalactone E pour son utilisation pour la prévention et/ou le traitement de cancers épithéliaux choisis parmi les mélanomes, les cancers du sein, de la prostate, de l'ovaire, du rein, de l'intestin, du colon, du pancréas, du poumon, de la thyroïde et les cancers ORL.

L'invention a également pour objet la Simalikalactone E pour son utilisation pour la prévention et/ou le traitement de cancers hématologiques choisis parmi les hémopathies malignes, et plus particulièrement les Syndromes MyéloProlifératifs (SMP) dont la Leucémie Myéloïde Chronique (LMC), les Syndromes MyéloDysplasiques (SMD), le Myélome Multiple (MM), les Leucémies Aiguës Myéloïdes (LAM), les Leucémies Aiguës Lymphoblastiques (LAL), l'utilisation de Simalikalactone E convenant plus particulièrement pour la prévention et/ou le traitement de la Leucémie Myéloïde Chronique (LMC), des Syndromes MyéloDysplasiques (SMD), du Myélome Multiple (MM), des Leucémies Aiguës Lymphoblastiques (LAL).

Les formes d'administration appropriées incluent les formes administrables par voie orale telles que les comprimés, les gélules molles ou dures, les poudres, les granules et les solutions ou suspensions orales, par voie topique par exemple sous forme de crèmes, gels, pommades ou lotions, par voie intraveineuse, par voie sous-cutanée, par voie intramusculaire, sous forme de suppositoire, de collyre, de gel ophtalmique ou d'implant retard sous la peau.

On peut préparer une composition solide sous forme de comprimés, par mélange du principe actif, la Simalikalactone E, avec un ou plusieurs excipients pharmaceutiquement acceptables, tels que, par exemple, la gélatine, l'amidon, le lactose, le stéarate de magnésium, le talc, la silice, la gomme arabique, le mannitol, la cellulose microcristalline, l'hydroxypropyl-méthylcellulose, ou des composants analogues. On peut enrober les comprimés de saccharose, d'un dérivé cellulosique ou d'autres matières adaptées à l'enrobage. Les comprimés peuvent être réalisés par différentes techniques bien connues de l'homme du métier, telles que la compression directe, la granulation sèche, la granulation humide ou la fusion à chaud.

On peut également préparer une composition sous forme de gélules en mélangeant les ingrédients actifs avec un diluant et en versant le mélange obtenu dans des gélules molles ou dures.

Les doses journalières de Simalikalactone E sont avantageusement les suivantes : de 10 à 50 mg/kg/j de Simalikalactone E. En particulier, ces doses conviennent à une administration par voie orale.

II peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient au traitement.

Le médicament de l'invention est destiné à être administré pendant préférentiellement 1 à 60 jours consécutifs, et de préférence de 20 à 40 jours, en une ou plusieurs prises quotidiennes, de préférence une seule prise par jour.

Une composition pharmaceutique comprenant la Simalikalactone E en tant que principe actif en association avec un autre principe actif anticancéreux, et au moins un excipient pharmaceutiquement acceptable est également décrite. Les principes actifs anticancéreux associés à la Simalikalactone E sont choisis parmi les principes actifs bien connus de l'homme du métier en fonction de la pathologie anticancéreuse à traiter.

La Simalikalactone E peut être isolée à partir de feuilles matures de *Quassia amara* en employant un protocole qui comprend les étapes suivantes : les feuilles matures de *Quassia amara* sont broyées et extraites par du méthanol. Cet extrait est dissous dans un système biphasique à base de n-heptane, acétate d'éthyle, méthanol, eau. La phase inférieure est recueillie et son volume est réduit de moitié par évaporation sous pression réduite. Cette solution est extraite avec de l'acétate d'éthyle. L'acétate d'éthyle est évaporé. Le résidu obtenu est dissout dans du chloroforme et lavé à l'aide d'une solution aqueuse légèrement basique. La phase organique est recueillie, séchée et concentrée sous pression réduite. Cet extrait est dissout dans de l'acétate d'éthyle et lavé à l'eau. La phase organique est évaporée sous pression réduite et le résidu obtenu est élué par de l'acétate d'éthyle à travers une colonne de silice.

L'extrait obtenu est purifié suivant le protocole suivant : l'extrait est fractionné par chromatographie de partage centrifuge en utilisant un système Arizona H composé de n-heptane, acétate d'éthyle, méthanol, eau, en mode ascendant. Des échantillons de 25 mL sont recueillis. Les échantillons comprenant la Simalikalactone E sont rassemblés et purifiés par chromatographie sur colonne de silice éluée par des mélanges cyclohexane/acétate d'éthyle de polarité croissante. La Simalikalactone E est éluée par le mélange cyclohexane/acétate d'éthyle 50/50.

Outre les dispositions qui précèdent, l'invention comprend encore d'autres dispositions qui ressortiront du complément de description qui suit, qui se rapporte à des exemples mettant en évidence les propriétés anti-tumorales de la Simalikalactone E, ainsi qu'aux dessins annexés dans lesquels :
- la **Figure 1** représente l'effet de la Simalikalactone E sur la croissance et la viabilité de lignées cellulaires leucémiques K562-Ima-S et K562-Ima-R,
- la **Figure 2** représente l'effet de la Simalikalactone E sur la croissance et la viabilité de cellules de mélanomes A375 et MeWo,
- la **Figure 3** met en évidence l'effet de la SkE sur la capacité clonogénique de cellules K562 (photographies prises avec un appareil numérique Canon®),
- la **Figure 4** représente l'effet de la Simalikalactone E sur la viabilité de cellules primaires humaines CD34+ de patients atteints de LMC,
- les **Figure 5** montre l'effet cinétique de la Simalikalactone E sur les marqueurs des différents types de mort : apoptotique (clivage de PARP) et autophagique (conversion de LC3). Le niveau de phosphorylation de la kinase Erk1/2 (MPA kinase) est également observé,
- la **Figure 6** montre l'effet de la Simalikalactone E sur la phosphorylation de la kinase Erk1/2 (MPA kinase),
- les **Figures 7** **et** **8** montrent l'effet de la Simalikalactone E sur des xénogreffes de cellules K562-Luc dans des souris Nude (**Figure 7** : quantification absolue du nombre de photons émis en valeur absolue (cpm = coup par minute) pour le PBS (Phosphate Buffered Saline), Ima (imatinib 60 mg/kg) et SkE (Simalikalactone E 1 mg/kg) ; **Figure 8** **:** acquisition réalisée grâce à un photo-imageur de type Biospace avec une acquisition de 5 min (**A** : souris traitées avec du PBS, **B :** souris traitées avec de l'imatinib 60 mg/kg et C : souris traitées avec de la SkE 1 mg/kg)).

### PARTIE EXPERIMENTALE

### A- Protocole d'obtention de la Simalikalactone E

Les feuilles de *Quassia amara* ont été récoltées à Rémire-Montjoly en Guyane Française. Un spécimen (GB3012) a été récolté et son identification botanique a été confirmée à l'herbier de Cayenne en Guyane.

La Simalikalactone E a été isolée de *Quassia amara* en employant le protocole suivant : 1 kg de feuilles matures séchées de *Quassia amara* sont broyées et extraites durant 24 heures avec 6 L de méthanol, ceci deux fois, pour donner 200 g d'extrait. 50 g de cet extrait sont dissous dans 2 L d'un système biphasique composé de n-heptane, acétate d'éthyle, méthanol, eau dans les proportions 3/2/3/2. La phase la plus lourde est concentrée jusqu'à 500 mL, et 500 mL d'eau y sont ajoutés. Cette solution est extraite avec 1 L d'acétate d'éthyle. L'acétate d'éthyle est évaporé sous pression réduite. Le résidu obtenu est dissous dans 1,5 L de chloroforme et lavé quatre fois à l'aide d'une solution d'hydroxyde de sodium 0,001 M. La phase organique est recueillie, séchée sur sulfate de magnésium anhydre et concentrée sous pression réduite pour donner 3,3 g d'extrait. Cet extrait est dissout dans 500 mL d'acétate d'éthyle et lavé avec 500 mL d'eau. La phase organique est évaporée sous pression réduite et le résidu obtenu est élué rapidement par l'acétate d'éthyle à travers une courte colonne de silice. On obtient après évaporation de l'acétate d'éthyle 1,0 g d'extrait. Cet extrait est fractionné par chromatographie de partage centrifuge (appareil Kromaton, équipé d'un rotor de 1 L), en utilisant un système Arizona H composé de n-heptane, acétate d'éthyle, méthanol, eau dans les proportions 1/3/1/3, en mode ascendant, à une température de 16°C. Le flux de solvant est de 25 mL/min, la vitesse du rotor est de 1000 t/min, et est changée au cours de l'élution pour maintenir une pression de 40 bars. L'extrait (1,0 g) est dissout dans 40 mL de système Arizona H, filtré et injecté. Des échantillons de 25 mL sont recueillis. Les échantillons sont rassemblés pour donner la fraction F1 (120 mg). Cette fraction est purifiée par chromatographie sur colonne de silice éluée par des mélanges cyclohexane/acétate d'éthyle de polarité croissante. La Simalikalactone E (10 mg) est éluée par le mélange cyclohexane /acétate d'éthyle 50/50. Le rendement à partir de la plante est de 0,004%.

La Simalikalactone E a été caractérisée par spectrométrie de masse, spectroscopie RMN, infrarouge, et par son pouvoir rotatoire. Des cristaux ayant été obtenus à partir du méthanol deutéré, un cliché de rayon X de la molécule a également été obtenu. Ses caractéristiques physico-chimiques sont les suivantes :
APCIMS, 579 (MH⁺), 561 (MH⁺-H₂O).
¹H NMR (CDCl₃, 500 MHz), δ 6,19 (m, 1H, H-15), 6,17 (s, 1H, H-3), 5,19 (dd, *J* = 2,6, 11,7 Hz, 1H, H-6), 4,75 (d, *J* = 5,1 Hz, 1H, H-11), 4,70 (d, *J* = 2,6 Hz, 1H, H-7), 4,65 (d, *J* = 7,4 Hz, 1H, H-17a), 4,19 (s, 1H, H-1), 3,83 (s, 1H, H-12), 3,70 (d, *J* = 7,4 Hz, 1H, H-17b), 3,37 (d, *J* = 11,5 Hz, 1H, H-5), 2,51 (m, 1H, H-24), 2,48 (m, 1H, H-19), 2,45 (m, 1H, H-14), 2,43 (m, 1H, H-9), 2,08 (s, 3H, H-30), 1,79 (m, 2H, H-21a, H-26a), 1,61 (m, 1H, H-26b), 1,53 (m, 1H, H-21b), 1,45 (s, 3H, H-28), 1,35 (s, 3H, H-29), 1,21 (d, *J* = 7,0 Hz, 3H, H-20), 1,19 (d, *J* = 7,0 Hz, 3H, H-25), 1,01 (t, *J* = 7,4 Hz, 3H, H-22), 0,99 (t, *J* = 7,4 Hz, 3H, H-27).
¹³C NMR (CDCl₃, 125 MHz), δ 196,5 (C-2), 176,2 (C-23), 175,2 (C-18), 166,5 (C-16), 163,0 (C-4), 126,5 (C-3), 82,8 (C-7), 81,8 (C-1), 80,0 (C-13), 79,8 (C-12), 74,2 (C-11), 70,9 (C-17), 69,1 (C-6), 67,3 (C-15), 52,7 (C-14), 50,4 (C-10), 46,1 (C-8), 45,9 (C-5), 41,3 (C-24), 41,3 (C-19), 41,1 (C-9), 27,2 (C-26), 26,7 (C-21), 26,1 (C-30), 22,8 (C-28), 16,7 (C-20), 15,6 (C-25), 12,5 (C-29), 11,7 (C-22), 11,5 (C-27).
IR (KBr, cm⁻¹) : 2965, 2926, 2855, 1766, 1738, 1722,1667.
[α]D²⁶ = +94° (c = 0,35, CHCl₃).

### B- Activité biologique

### B1- Matériel et Méthodes

Les drogues utilisées sont prépalablement diluées 1000 fois dans du DMSO pur (concentration finale en DMSO : 0,1%).

### B1-1. Mesure de viabilité cellulaire XTT

Ce test mesure l'activité de la succinate déshydrogénase mitochondriale qui représente un index de la viabilité cellulaire. Cet enzyme transforme le XTT en formazan orange. Au jour 0, les cellules K562-Ima-S (sensibles à Ima), K562-Ima-R (résistantes à Ima), A375 ou MeWo sont ensemencées à raison de 15 000 cellules/puits (100 µL) dans des microplaques de 96 puits et stimulées avec des concentrations croissantes de Simalikalactone E [50-500 nM]. Après 24 ou 48 heures, 50 µL de tétrazolium (réactif XTT) sont ajoutés à chaque puits et une lecture de la densité optique à 490 nm est réalisée toutes les 30 minutes pendant 2 à 4 heures. Les valeurs obtenues qui sont un index du métabolisme mitochondrial permettent d'évaluer le pourcentage de cellules vivantes. Chaque condition est systématiquement réalisée en triplicate.

### B1-2. Evaluation de la capacité clonogénique de cellules K562

Des aliquots de méthylcellulose (40% méthocult®, 1% L-Glutamine, 5% SVF (sérum de veau foetal) et 54% IMDM (Iscove's modified Dulbecco's medium)) sont chauffés pendant 1h à 37°C. A chaque aliquot est ajouté 400 µL de RPMI (Roswell Park Memorial Institute médium), 5% de SVF contenant le traitement (imatinib 1µM, SkE 100, 250 ou 500 nM), puis 10³ cellules K562 dans 200 µL de milieu. Pour chaque condition, 500 µL de la suspension obtenue est alors déposé dans 3 puits d'une plaque 24 puits. Une condition non traité sert de contrôle. Les plaques sont incubées à 37°C pendant 7 à 10 jours. 75 µL de sel de tétrazolium MTT (Roche) (2,5 mg/ml) sont ajoutés à chaque puits. Le sel de tétrazolium MTT est clivé en cristaux de formazan de couleur violette par les cellules métaboliquement actives. Après 4h d'incubation à 37°C, les puits sont photographiés et les clones quantifiés grâce au programme Image J.

### B1-3. Mesure de la mort cellulaire

La caractérisation du type de mort cellulaire impliqué dans l'effet de la Simalikalactone E est réalisée selon les protocoles suivants :
- l'apoptose (mort cellulaire de type I) est étudiée grâce à des dosages d'activité caspases, protéases à cystéines impliquées dans ce type de processus. L'apoptose est également quantifiée par co-marquage annexine V/iodure de propidium, les cellules externalisant l'annexine V au niveau de leur mitochondrie étant des cellules en cours d'apoptose,
- la mort autophagique (mort cellulaire de type II) est analysée par des dosages d'activité cathepsines, ces protéases étant quant à elles impliquées dans la dégradation du matériel intracellulaire inclus dans les vésicules (autophagolysosomes). La mort cellulaire de type II peut également être mise en évidence par imagerie en microscopie électronique, et
- des westerns blot (transferts de protéines) sont enfin réalisés en utilisant des anticorps spécifiques de l'induction d'apoptose (clivage de PARP et des caspases) ou de la mort autophagique (conversion de LC3-I en LC3-II et activation des cathepsines).

### B1-4. Xénogreffes de cellules K562-Luc sur des souris Nude

La lignée utilisée pour les xénogreffes est une lignée de K562-Luc exprimant de manière stable le gène codant pour la luciférase. Elle est cultivée dans un milieu RPMI 1640 contenant 5% de SVF, supplémenté en pyruvate de sodium et en Pénicilline/Streptomycine et contenant 10 µM de Blastycidine (antibiotique permettant la sélection des cellules possédant le plasmide codant pour la luciférase).

Pour chaque injection, 10⁶ cellules K562-Luc sont préparées dans 200 µL de milieu, 70% PBS et 30% matrigel. Chaque souris Nude reçoit 1 injection sous-cutanée sur chaque flanc. Les souris reçoivent 3 fois par semaine une injection de 100 µL en intrapéritonéale. Selon les groupes, les animaux reçoivent soit du PBS, soit de l'imatinib (60 mg/kg), soit de la SkE (1 mg/kg).

L'évaluation de la croissance tumorale est réalisée 1 fois par semaine.

Chaque animal éveillé reçoit une injection de 100 µL de Luciférine à 30 mg/mL (Calliper LifeSciences). Après 5 min, les animaux sont anesthésiés par inhalation de 5% d'isoflurane par minute et par litre d'O₂. Les animaux sont alors transférés par groupe de 5 dans le photon imager où l'anesthésie est maintenue avec 2% d'isoflurane. La luciférine est dégradée par la luciférase exprimée par les cellules tumorales. Cette dégradation s'accompagne de l'émission de photons mesurés par le photon Imager.

Grâce à l'intensité de l'émission de photons on peut évaluer la taille et l'activité métabolique de la tumeur.

### B2- Résultats

### B2-1. Inhibition de la croissance et réduction de la viabilité de différences lignée tumorales cellulaires

Les lignées tumorales K562-Ima-S et K562-Ima-R sont incubées pendant 48 heures avec des concentrations croissantes de la Simalikalactone E (0, 100 ou 250 nM) en absence ou en présence d'une concentration maximalement efficace (50 µM) de z-Vad-fmk (Zvad) un inhibiteur pan-caspase (inhibiteur d'apoptose).

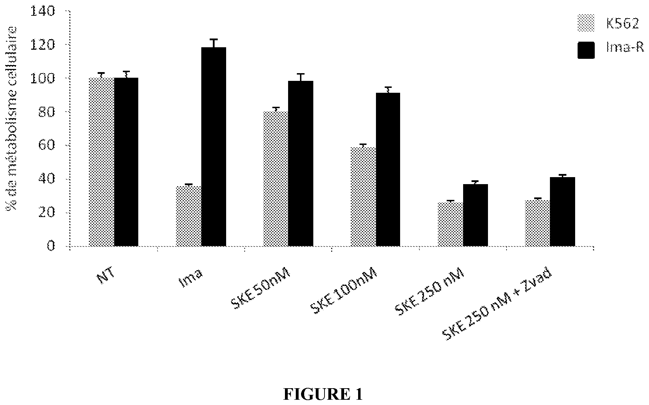

Les résultats sont présentés à la **Figure 1****.**

Les deux lignées leucémiques sont sensibles à l'effet de la Simalikalactone E et l'effet de la Simalikalactone E n'est pas modifié en présence de l'inhibiteur de caspase, ce qui démontre que la perte de viabilité des cellules en présence de Simalikalactone E ne requiert pas d'apoptose.

Des cellules de mélanomes A375 et MeWo sont ensuite traitées en présence de doses croissantes de Simalikalactone E. 48 heures plus tard, les cellules sont soumises à un test XTT. Les cellules A375 sont porteuses de la mutation V600E sur l'oncogène B-raf ce qui se traduit par une activation constitutive de la voie Erk. De façon intéressante les cellules A375 sont beaucoup plus sensibles à la Simalikalactone E que les cellules MeWo qui ne possèdent pas la mutation de B-raf.

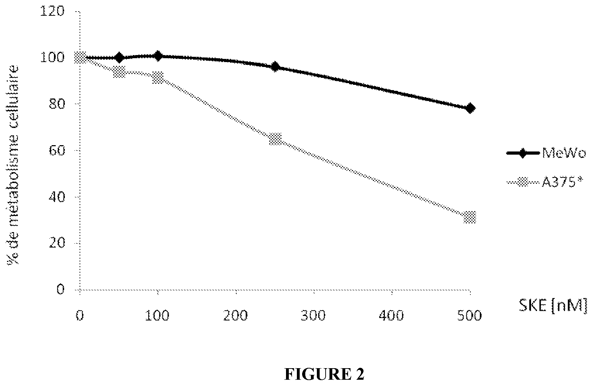

Les résultats sont présentés à la **Figure 2****.**

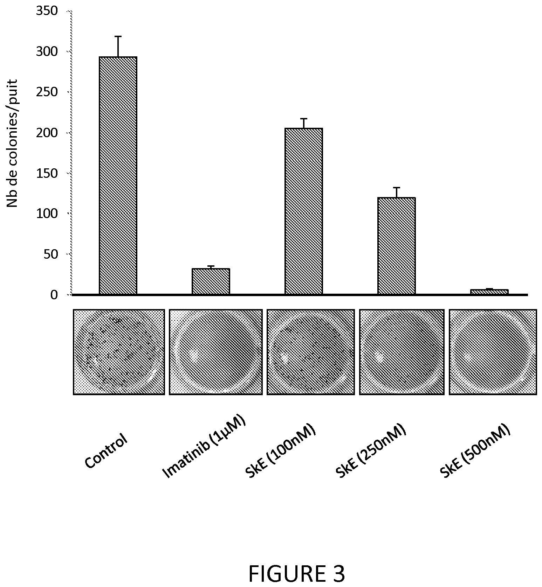

L'effet de la SkE sur la capacité clonogénique de cellules K562 a également été mis en évidence sur la **Figure 3****.** La capacité clonogénique représente la capacité à former une colonie à partir d'une cellule isolée, propriété inhérente aux cellules cancéreuses. On observe ainsi que la SkE est capable d'inhiber la capacité clonogénique de cellules K562 de manière aussi efficace que l'imatinib (traitement de référence de la pathologie LMC) qui agit directement sur la protéine chimérique BCR-ABL, et ce à des concentrations 2 a 4 fois inferieures.

### B2-2. Inhibition de la viabilité de cellules primaires humaines CD34+ de patients atteint de LMC

Les cellules primaires de patients atteints de LMC (CD34+ Bcr-Abl+ purifiées) sont incubées en présence de concentrations variables de Simalikalactone E pendant 48 heures, puis la viabilité cellulaire est quantifiée grâce à un test colorimétrique XTT. L'imatinib (Ima) est utilisé comme contrôle positif de l'induction de mort des cellules Bcr-Abl+.

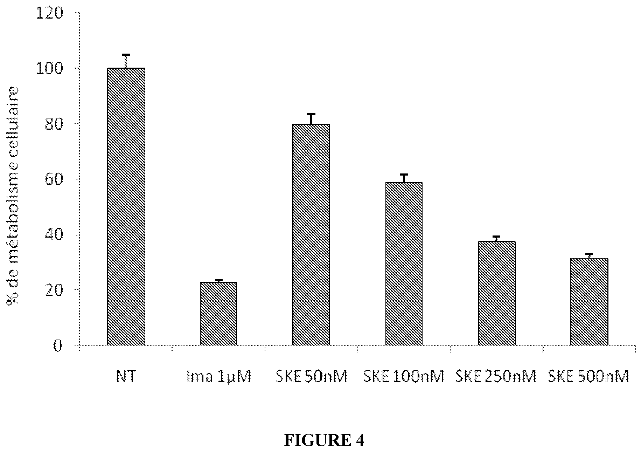

Les résultats sont présentés à la **Figure 4****.**

La Simalikalactone E à une concentration de 500 nM s'avère aussi efficace que l'imatinib pour inhiber le métabolisme cellulaire. L'effet demi-maximal est obtenu pour une dose de 100 nM de Simalikalactone E.

### B2-3. Inhibition de la phosphorylation de la kinase Erk1/2 (MPA kinase)

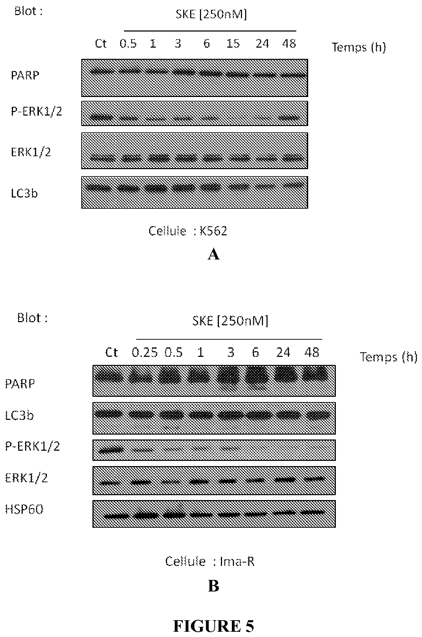

Les cellules K562-Ima-S (A) et K562-Ima-R (B) sont traitées pendant des durées de temps variables avec 250 nM de Simalikalactone E. Les cellules sont ensuite lysées et des extraits protéiques sont préparés pour une analyse en western blot. Enfin, les membranes sont incubées en présence des différents Anticorps anti-PARP, LC3b, p-Erk1/2, Erk1/2 et HSP60. La **Figure 5** montre que la Simalikalactone E inhibe la phosphorylation de la kinase Erk1/2 (MPA kinase) aussi bien dans les cellules sensibles que résistantes à l'imatinib, sans modifier l'expression globale de la protéine Erk. Cet effet de la Simalikalactone E se produit en absence d'apoptose (pas de clivage de PARP) et d'autophagie (pas de modification de LC3).

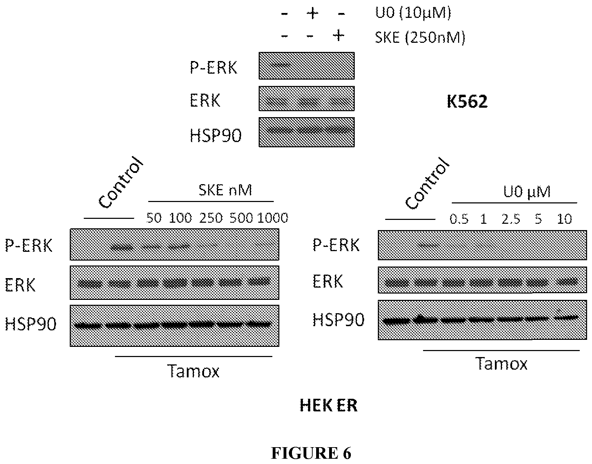

Des cellules K562-Ima-S sont ensuite traitées pendant 1 heure en présence de 250 nM de Simalikalactone E ou 10 µM d'U0126 (inhibiteur spécifique de MEK1/2, la kinase responsable de la phosphorylation et de l'activation de l'Erk1/2 (MPA kinase)). Les cellules sont ensuite lysées et des extraits protéiques préparés pour une analyse en western blot. Les membranes sont alors incubées en présence des anticorps p-Erk1/2, Erk1/2 et HSP60. La **Figure 6A** montre que la Simalikalactone E à une concentration de 250 nM est aussi efficace que l'U0126 à une concentration 40 fois plus forte, l'U0126 étant l'inhibiteur de MEK1/2 actuellement le plus utilisé pour bloquer l'activité de l'Erk1/2 (MPA kinase) *in vitro.*

Dans l'expérience présentée à la **Figure 6B****,** des cellules HEK-Raf1-ER inductibles ont été utilisées. Ces cellules expriment la kinase Raf sous le contrôle du récepteur des oestrogènes. L'expression de Raf peut donc être induite en présence de tamoxifène. Les cellules sont pré-incubées pendant 30 minutes avec des doses croissantes de Simalikalactone E ou d'U0126, puis traitées avec 1 µM de tamoxifène pendant 1 heure. Les cellules sont ensuite lysées et des extraits protéiques sont préparés pour une analyse en western blot. Les membranes sont finalement incubées en présence des différents anticorps (p-Erk1/2, Erk1/2 et HSP60). La Simalikalactone E et l'U0126 inhibent la phosphorylation de l'Erk1/2 (MPA kinase) dépendante de Raf1. Ces résultats montrent que la Simalikalactone E bloque la voie Erk1/2 (MPA kinase) au niveau ou en amont de MEK1/2.

### B2-4. Xénogreffes de cellules K562-Luc dans des souris Nude

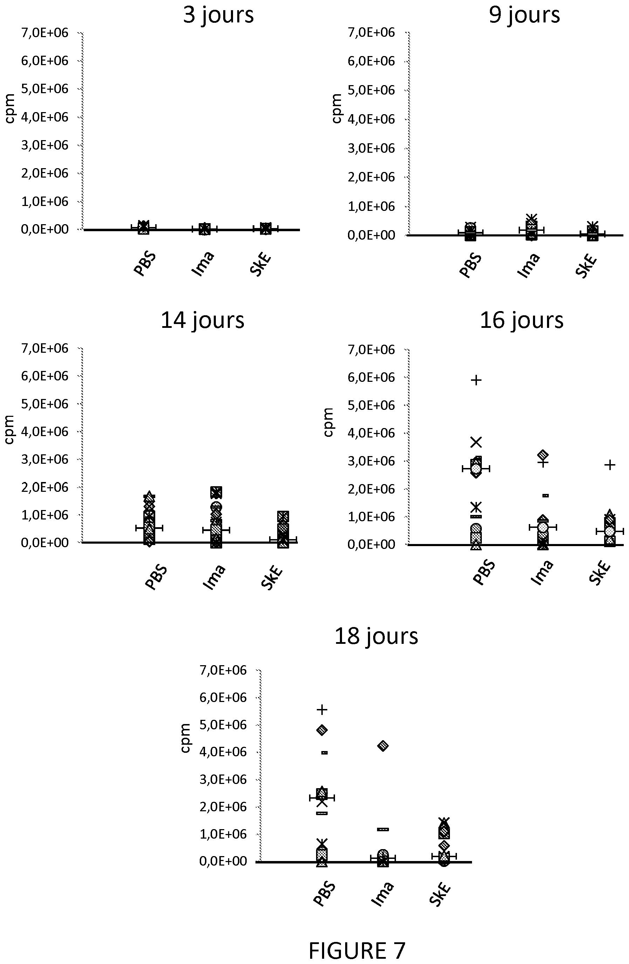

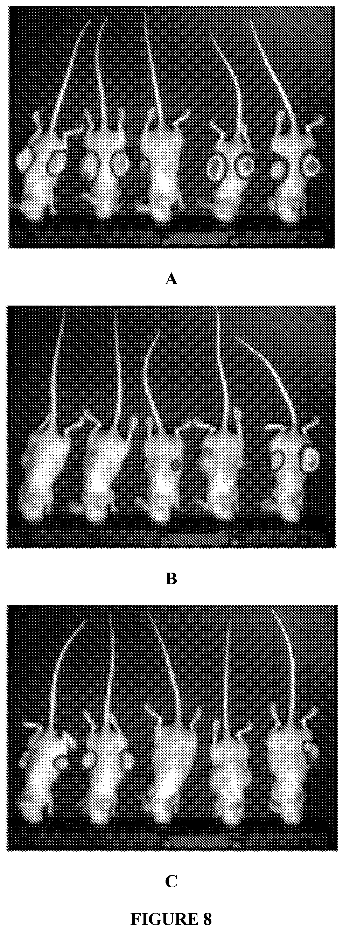

Les résultats des xénogreffes de cellules K562-Luc sur des souris Nude sont présentés aux **Figures 7** **et** **8****.**

La **Figure 8** montre que les souris traitées avec le PBS (traitement contrôle) présentent d'importantes tumeurs, en comparaison aux souris traitées avec l'imatinib 60 mg/kg (traitement de référence) et aux souris traitées avec la SkE 1 mg/kg.

## Revendications

1. Simalikalactone E répondant à la formule 1 ci-dessous : 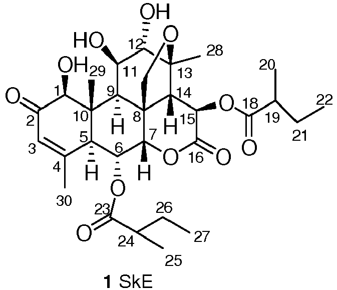 pour son utilisation pour la prévention et/ou le traitement de cancers épithéliaux et hématologiques dans lesquels la voie de signalisation Ras/Raf/MEK/Erk est activée.

2. Simalikalactone E pour son utilisation selon la revendication 1, pour la prévention et/ou le traitement de cancers épithéliaux choisis parmi les mélanomes, les cancers du sein, de la prostate, du pancréas, de l'ovaire, du rein, de l'intestin, du colon, du poumon, de la thyroïde et les cancers ORL.

3. Simalikalactone E pour son utilisation selon la revendication 2, pour la prévention et/ou le traitement de mélanomes.

4. Simalikalactone E pour son utilisation selon la revendication 1, pour la prévention et/ou le traitement de cancers hématologiques choisis parmi les hémopathies malignes.

5. Simalikalactone E pour son utilisation selon la revendication 4 , pour la prévention et/ou le traitement des Syndromes MyéloProlifératifs (SMP), dont la Leucémie Myéloïde Chronique (LMC), les Syndromes MyéloDysplasiques (SMD), le Myélome Multiple (MM), les Leucémies Aiguës Myéloïdes (LAM), les Leucémies Aiguës Lymphoblastiques (LAL).

6. Simalikalactone E pour son utilisation selon la revendication 5, pour la prévention et/ou le traitement de la Leucémie Myéloïde Chronique (LMC), des Syndromes MyéloDysplasiques (SMD), du Myélome Multiple (MM), des Leucémies Aiguës Lymphoblastiques (LAL).

7. Simalikalactone E pour son utilisation selon l'une des revendications 1 à 6, dans laquelle la Simalikalactone E se présente sous une forme administrable par voie orale, topique, intraveineuse ou sous-cutanée.

8. Simalikalactone E pour son utilisation selon l'une des revendications 1 à 7, dans laquelle la dose journalière de Simalikalactone E varie de 10 à 50 mg/kg/j.

9. Simalikalactone E pour son utilisation selon l'une des revendications 1 à 8, dans laquelle la durée de traitement est comprise entre 1 et 60 jours consécutifs.

## Patentansprüche

1. Simalikalacton E der nachstehenden Formel 1: 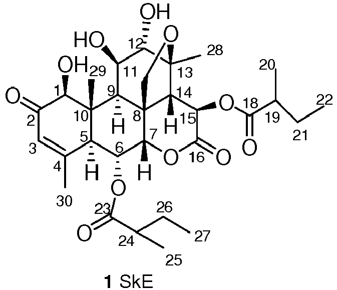 für die Verwendung zur Vorbeugung und/oder Behandlung epithelialer und hämatologischer Krebserkrankungen, bei denen der Ras/Raf/MEK/Erk-Signalweg aktiviert ist.

2. Simalikalacton E für die Verwendung nach Anspruch 1 zur Vorbeugung und/oder Behandlung epithelialer Krebserkrankungen, ausgewählt aus Melanomen, Brust-, Prostata-, Bauchspeicheldrüsen-, Eierstock-, Nieren-, Darm-, Kolon-, Lungen-, Schilddrüsen- und HNO-Krebserkrankungen.

3. Simalikalacton E für die Verwendung nach Anspruch 2 zur Vorbeugung und/oder Behandlung von Melanomen.

4. Simalikalacton E für die Verwendung nach Anspruch 1 zur Vorbeugung und/oder Behandlung hämatologischer Krebserkrankungen, ausgewählt aus malignen Hämatopathien.

5. Simalikalacton E für die Verwendung nach Anspruch 4 zur Vorbeugung und/oder Behandlung von myeloproliferativen Syndromen (SMP), einschließlich chronischer myeloischer Leukämie (CML), myelodysplastischen Syndromen (MDS), Multiplem Myelom (MM), akuten myeloischen Leukämien (AML), akuten lymphatischen Leukämien (ALL).

6. Simalikalacton E für die Verwendung nach Anspruch 5 zur Vorbeugung und/oder Behandlung von chronischer myeloischer Leukämie (CML), myelodysplastischen Syndromen (MDS), Multiplem Myelom (MM), akuten lymphatischen Leukämien (ALL).

7. Simalikalacton E für die Verwendung nach einem der Ansprüche 1 bis 6, wobei das Simalikalacton E in einer oral, topisch, intravenös oder subkutan verabreichbaren Form vorliegt.

8. Simalikalacton E für die Verwendung nach einem der Ansprüche 1 bis 7, wobei die tägliche Dosis von Simalikalacton E von 10 bis 50 mg/kg/Tag variiert.

9. Simalikalacton E für die Verwendung nach einem der Ansprüche 1 bis 8, wobei die Behandlungsdauer zwischen 1 und 60 aufeinanderfolgende Tage beträgt.

## Claims

1. Simalikalactone E corresponding to formula 1 below: 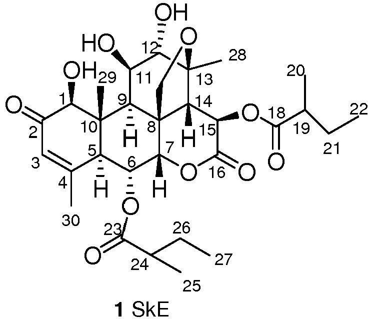 for the use thereof for preventing and/or treating epithelial and hematological cancers in which the Ras/Raf/MEK/Erk signaling pathway is activated.

2. Simalikalactone E for the use thereof according to Claims 1, for preventing and/or treating epithelial cancers chosen from melanomas, breast, prostate, pancreatic, ovarian, kidney, intestinal, colon, lung and thyroid cancers and ENT cancers.

3. Simalikalactone E for the use thereof according to Claim 2, for preventing and/or treating melanomas.

4. Simalikalactone E for the use thereof according to Claim 1, for preventing and/or treating hematological cancers chosen from malignant hemopathies.

5. Simalikalactone E for the use thereof according to Claim 4, for preventing and/or treating myeloproliferative syndromes (MPS), including chronic myeloid leukemia (CML), myelodysplastic syndromes (MDS), multiple myeloma (MM), acute myeloid leukemias (AML) and acute lymphoblastic leukemias (ALL).

6. Simalikalactone E for the use thereof according to Claim 5, for preventing and/or treating chronic myeloid leukemia (CML), myelodysplastic syndromes (MDS), multiple myeloma (MM) and acute lymphoblastic leukemias (ALL).

7. Simalikalactone E for the use thereof according to one of Claims 1 to 6, wherein the Simalikalactone E is in a form which is administrable orally, topically, intravenously or subcutaneously.

8. Simalikalactone E for the use thereof according to one of Claims 1 to 7, wherein the daily dose of Simalikalactone E ranges from 10 to 50 mg/kg/d.

9. Simalikalactone E for the use thereof according to one of Claims 1 to 8, wherein the duration of treatment is between 1 and 60 consecutive days.
